# EUROPEAN PATENT APPLICATION

(11) **EP 4 653 027 A1**
(43) Date of publication of application: **26.11.2025**
(21) Application number: 24744925.9
(22) Date of filing: 19.01.2024
(51) Int. Cl.: A61M 1/36

(54) **EXTRACORPOREAL BLOOD PURIFICATION DEVICE THROUGH REMOVAL OF REACTIVE OXYGEN SPECIES AND EXTRACORPOREAL BLOOD PURIFICATION METHOD USING SAME**

(30) Priority: 19.01.2023 KR 20230007902
(71) Applicant: Eternox Inc., Seongnam-si, Gyeonggi-do 13605 (KR)
(72) Inventor: JEONG, Han-Gil, Seoul 05673 (KR); KIM, Jaeyun, Suwon-si, Gyeonggi-do 16419 (KR); IM, Pilseon, Seoul 07600 (KR); KIM, Dong Hee, Osan-si, Gyeonggi-do 18133 (KR)
(74) Representative: Dentons Patent Solutions Rechtsanwaltsgesellschaft mbH
(86) International application number: PCT/KR2024/000964
(87) International publication number: WO 2024/155146

(57) **Abstract**

The present specification pertains to an extracorporeal blood purification device and an extracorporeal blood purification method using same. In an aspect, the present invention can effectively remove reactive oxygen species (ROS) from blood, which are substances causative of inflammation, using porous microbeads loaded with ceria nanoparticles. Specifically, the extracorporeal blood purification device is manufactured to allow a patient's blood to perfuse through a cartridge having the porous microbeads supported thereon, enabling the rapid and extensive removal of ROS in aqueous and blood conditions. Therefore, the present invention can be applied to microbead-based extracorporeal blood perfusion therapy systems.

## Description

### Technical Field

Disclosed herein are an extracorporeal hemopurification device and an extracorporeal hemopurification therapy using the same.

Meanwhile, this application was supported by the following research and development projects.

### [Research and development projects supporting this invention]

[Task Number] 2022R1C1C100961012
[Ministry Name] Ministry of Science and ICT
[Management Agency] National Research Foundation of Korea
[Project Name] Basic Research Project in Science and Engineering > New Research Support Project > Excellent New Research
[Task Title] Development of an Extracorporeal Hemopurification System for the Treatment of Sepsis Based on Multifunctional Microbead
[Contribution Rate] 60/100
[Leading Institution] Seoul National University Bundang Hospital
[Research Period] March 1, 2022 - February 28, 2025
[Task Number] RS-2023-00222910
[Ministry Name] Ministry of Science and ICT
[Management Agency] National Research Foundation of Korea
[Project Name] Development of Bio and Medical Technologies (R&D)
[Task Title] Development of Application Technologies for the Five Major Diseases and Cultivation of Physician-Scientists through a Future Medical Research Center Tailored for the Era of 6P Medicine
[Contribution Rate] 35/100
[Leading Institution] Seoul National University Bundang Hospital
[Research Period] April 1, 2023 - December 31, 2026
[Task Number] 13-2021-0004
[Ministry Name] Seoul National University Bundang Hospital
[Management Agency] Seoul National University Bundang Hospital
[Project Name] Joint Research in Key Support Areas
[Task Title] Development of an Extracorporeal Hemopurification Device for Treating Cytokine Storms Based on Nanomaterials
[Contribution Rate] 5/100
[Leading Institution] Seoul National University Bundang Hospital
[Research Period] March 1, 2021 - March 1, 2024

### Background Art

Sepsis is a severe infectious condition accompanied by an essentially systemic inflammatory response. Sepsis may lead to tissue damage and organ dysfunction and progress to life-threatening conditions due to excessive and erroneous reactions of the human body to infection. The systemic inflammatory response syndrome (SIRS) is a systemic inflammatory response that occurs as the body reacts to various forms of severe stress or injury, such as infection, trauma, burns, or pancreatitis, and it plays an important pathophysiological role in sepsis. To date, a variety of drugs have been developed and clinically tested for the treatment of sepsis but have failed to demonstrate effectiveness. As a new approach to treating sepsis, hemopurification therapies that remove pathogen-associated substances or cytokines extracorporeally (e.g., Toraymyxin, CytoSorb, etc.) have been developed and generated expectations but have not succeeded in clinical trials, and their use is not recommended in clinical guidelines. Reactive oxygen species are critical factors that are excessively generated or inadequately removed in systemic inflammatory response syndrome, including sepsis, leading to cell damage and promoting inflammation that triggers tissue injury and organ dysfunction. However, there is a clear limitation in existing hemopurification therapies as they are unable to effectively remove reactive oxygen species.

### Detailed Description of the Invention

### Technical Problem

One objective of the present disclosure is to provide porous microbeads for extracorporeal hemopurification , an extracorporeal hemopurification cartridge including the porous microbeads, or an extracorporeal hemopurification device including the cartridge.

Another objective of the present disclosure is to provide a method for preparing the porous microbeads.

Another objective of the present disclosure is to provide a method for extracorporeal hemopurification using the porous microbeads.

### Solution to Problem

In order to achieve the above objectives, the present disclosure provides, in one aspect, porous microbeads for extracorporeal hemopurification , including ceria nanoparticles.

In an exemplary embodiment, the porous microbeads are capable of removing reactive oxygen species by catalysis of the ceria nanoparticles.

In another aspect, the present disclosure provides an extracorporeal hemopurification cartridge, including the porous microbeads.

In yet another aspect, the present disclosure provides an extracorporeal hemopurification device, including the extracorporeal hemopurification cartridge.

In yet another aspect, the present disclosure provides an extracorporeal hemopurification method, including the steps of:
(a) contacting blood isolated from a subject with the porous microbeads;
(b) removing reactive oxygen species from the blood using the porous microbeads; and
(c) collecting blood from which the reactive oxygen species have been removed.

In yet another aspect, the present disclosure provides a method for preparing the porous microbeads for extracorporeal hemopurification , including the steps of:
(a) synthesizing the porous microbeads and modifying their surface; and
(b) attaching ceria nanoparticles to the modified surface of the porous microbeads.

### Effects of the Invention

In an aspect, the present disclosure may effectively remove reactive oxygen species (ROS) from blood extracorporeally, which are substances causative of inflammation, using porous microbeads loaded with ceria nanoparticles. Specifically, the extracorporeal hemopurification device is prepared to allow a patient's blood to perfuse through a cartridge having the porous microbeads supported thereon, enabling the rapid and extensive removal of ROS in aqueous and blood conditions. Therefore, the present disclosure may be applied to a microbead-based extracorporeal hemopurification therapy system.

In another aspect, the present disclosure allows for sustained removal of reactive oxygen species (ROS). In order to solve the problem that continuous ROS removal is difficult particularly in an extracorporeal perfusion environment, the present disclosure may achieve sustained ROS removal capability by using a catalyst (ceria nanoparticles) instead of an adsorbent.

In yet another aspect, inorganic nanoparticles such as ceria nanoparticles are difficult to develop as drugs because they are difficult to ensure safety when injected into the body, but the present disclosure has the advantage of ensuring safety because the blood is perfused outside the body to remove ROS.

In yet another aspect, the present disclosure may solve the problems of existing extracorporeal hemopurification devices because it may selectively remove ROS, which is a fundamental problem of inflammation-damaging reactions, unlike existing extracorporeally perfused therapies, which have non-specifically adsorbed even substances (e.g., cytokines) that may be beneficial to the patient.

In yet another aspect, the present disclosure may eliminate increased ROS in severe diseases associated with systemic inflammatory response syndrome, including sepsis, through extracorporeal perfusion, improve severity indicators (e.g., blood pressure, blood pressure enhancer usage, lactate levels), and improve survival rate.

In yet another aspect, the present disclosure may be applied to a microbead-based extracorporeal hemopurification therapy system that removes reactive oxygen species (ROS), which are substances causative of inflammation.

### Brief Description of the Drawings

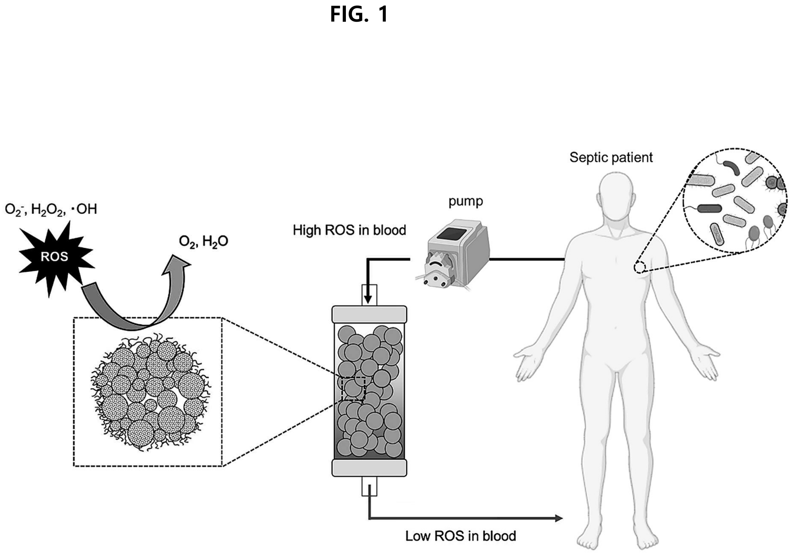

FIG. 1 is a schematic view of a microbead-based hemopurification system for the removal of reactive oxygen species in a sepsis model.

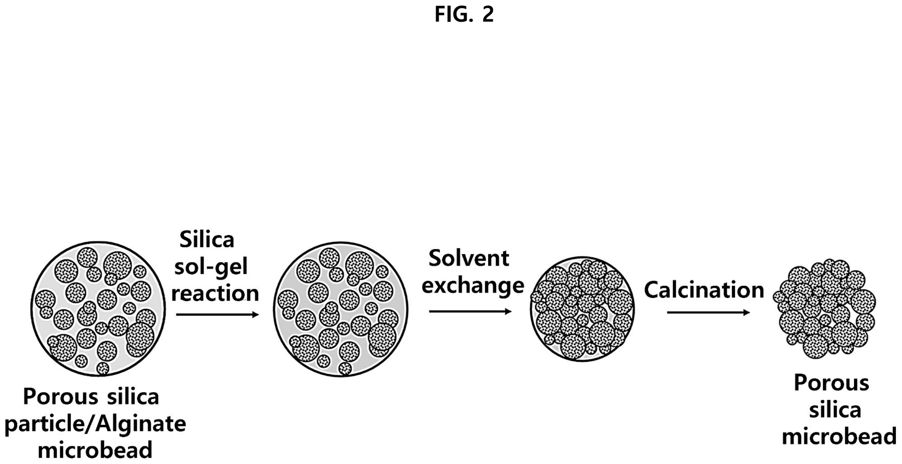

FIG. 2 relates to a process for preparing porous silica microbeads.
FIG. 3 is a representative image of each step in the preparation of porous silica microbeads from porous silica particles/alginate microbeads.
FIG. 4 relates to the nitrogen (N₂) physisorption isotherms and pore diameter (square: 1 wt%, triangle: 3 wt%, diamond: 5 wt%) of the final product, porous silica microbeads, as a function of the content of porous silica particles (wt%) during the fabrication of the initial porous silica particles/alginate microbeads.
FIG. 5 relates to a process for fabricating ceria nanoparticles/porous silica microbeads by attaching ceria nanoparticles with electrostatic attraction to the surface of porous silica microbeads.
FIG. 6 relates to the surface charge (zeta-potential) of porous silica particles and ceria nanoparticles.
FIG. 7 is a representative photographic image of porous silica microbeads and ceria nanoparticles/porous silica microbeads with attached ceria nanoparticles.
FIG. 8 relates to SEM images of porous silica microbeads and ceria nanoparticles/porous silica microbeads, along with the analysis of the atomic distribution of cerium (Ce) on the surface of the microbeads using EDS.
FIG. 9 is a schematic diagram of ceria nanoparticles/porous silica microbeads coated with PVP on the surface.
FIG. 10 relates to thermogravimetric analysis (TGA) results of porous silica microbeads, ceria nanoparticles/porous silica microbeads, PVP-coated ceria nanoparticles/porous silica microbeads.
FIG. 11 relates to catalase-mimicking activity using porous silica microbeads, ceria nanoparticles/porous silica microbeads, PVP-coated ceria nanoparticles/porous silica microbeads by hydrogen peroxide/peroxidase analysis with incubation time in H₂O₂(500µM).
FIG. 12 relates to hydroxyl radical scavenging activity of porous silica microbeads, ceria nanoparticles/porous silica microbeads and PVP-coated ceria nanoparticles/porous silica microbeads using a HORAC assay kit.
FIG. 13 relates to catalase-mimicking activity as a function of the amount of ceria nanoparticles included in the microbeads.
FIG. 14 is a representative image of a cartridge fabricated for mounting microbeads.
FIG. 15 is a representative image of cartridges each loaded with porous silica microbeads, ceria nanoparticles/porous silica microbeads and PVP-coated ceria nanoparticles/porous silica microbeads.
FIG. 16 relates to catalase-mimicking activity when hydrogen peroxide is flowed into a cartridge loaded with 220 g each of porous silica microbeads, ceria nanoparticles/porous silica microbeads and PVP-coated ceria nanoparticles/porous silica microbeads, and the hydrogen peroxide passing through the cartridge is collected and repeatedly flowed up to 7 times.
FIG. 17 relates to catalase-mimicking activity measured after passing a fresh hydrogen peroxide solution through a cartridge loaded with PVP-coated ceria nanoparticles/porous silica microbeads and collecting the solution each time.
FIG. 18 shows the catalase-mimicking properties of a cartridge fabricated by loading ceria nanoparticles onto glass microbeads without pores and porous PES (polyethersulfone) polymer microbeads, compared to PVP-coated ceria nanoparticles/porous silica microbeads cartridges.
FIG. 19 is a representative image of microbeads centrifuged after mixing with blood to show the hemocompatibility of the microbeads.
FIG. 20 relates to the degree of hemolysis based on the absorbance analysis of the supernatant.
FIG. 21 is a representative image of centrifuged microbeads after washing away blood from the centrifuged microbeads.
FIG. 22 is an image captured in chronological order showing the blood being perfused from the body into the cartridge during actual extracorporeal hemopurification treatment.
FIG. 23 compares the differences in hydrogen peroxide (H₂O₂) levels released by white blood cells extracted from the blood of the group undergoing the hemopurification treatment and the control group in laboratory rats induced with sepsis.
FIG. 24 compares the differences in blood lactate levels, a marker of septic shock, between the group undergoing the hemopurification treatment and the control group in laboratory rats induced with sepsis.
FIG. 25 compares the difference in survival rate between the hemopurification treatment group and the control group in laboratory rats induced with sepsis.
FIG. 26 compares the differences in blood pressures between the hemopurification treatment group and the control group in laboratory rats induced with sepsis.
FIG. 27 compares the differences in the amount of vasopressor used, reflecting the degree of shock, in the hemopurification treated group and the control group in laboratory rats induced with sepsis.

### Best Mode for Carrying out the Invention

Hereinafter, the present disclosure will be described in detail.

In one aspect, the present disclosure relates to porous microbeads for extracorporeal hemopurification including ceria nanoparticles.

In an exemplary embodiment, the porous microbeads are capable of removing reactive oxygen species by catalysis of the ceria nanoparticles.

In an exemplary embodiment, the size (diameter) of the porous microbeads may be, but is not limited to, 1 µm or more, 10 µm or more, 100 µm or more, 150 µm or more, 200 µm or more, 250 µm or more, 300 µm or more, 350 µm or more, 400 µm or more, 450 µm or more, or 1500 µm or less, 1400 µm or less, 1300 µm or less, 1200 µm or less, 1,100 µm or less, 1000 µm or less, 900 µm or less, 800 µm or less, 700 µm or less, 650 µm or less, 600 µm or less, 550 µm or less. For example, the porous microbeads may have a size (diameter) of 1-1500 µm, or 10-1000 µm, or 100-1000 µm. The size (diameter) of the porous microbeads may be adjusted by taking into account the size of the cartridge and effective hemopurification . Furthermore, in a nonlimiting example, the size (diameter) of the porous microbeads may be such that they are larger than blood cells, e.g., greater than 10 µm.

In an exemplary embodiment, the reactive oxygen species may be one or more selected from the group consisting of, but are not limited to, superoxide anion, hydrogen peroxide, hydroxyl radical, and combinations thereof.

In an exemplary embodiment, the pore volume of the porous microbeads may be, but is not limited to, from 0.1 cm³/g to 13.5 cm³/g, preferably from 0.1 cm³/g to 5.0 cm³/g, and more preferably from 0.5 cm³/g to 1.5 cm³/g. The porous microbeads having such pore volumes may effectively load ceria nanoparticles and remove reactive oxygen species.

In an exemplary embodiment, the surface area of the porous microbeads may be, but is not limited to, from 100 m²/g to 1000 m²/g, preferably from 150 m²/g to 600 m²/g, and more preferably from 200 m²/g to 400 m²/g. The porous microbeads having such surface areas may effectively load ceria nanoparticles and remove reactive oxygen species.

In an exemplary embodiment, the porous microbeads may include, but are not limited to, 3 mg/g or more of ceria nanoparticles, specifically 3 mg/g or more, 4 mg/g or more, 5 mg/g or more, 10 mg/g or more, 15 mg/g or more, 20 mg/g or more, 30 mg/g or more, 40 mg/g or more, or 50 mg/g or more, or 500 mg/g or less, 400 mg/g or less, 300 mg/g or less. For example, porous microbeads in a cartridge containing 100 mg/g or 200 mg/g of ceria nanoparticles may effectively perform extracorporeal hemopurification and removal of reactive oxygen species.

In an exemplary embodiment, the porous microbeads are one or more selected from the group consisting of porous microbeads coated with Alginate, porous microbeads coated with polyethylene glycol(PEG), porous microbeads coated with polyvinylpyrrolidone (PVP), and combinations thereof. The coating may prevent reactivity of the porous microbeads with blood, thereby inhibiting hemolysis or agglutination, and consequently improving the hemocompatibility of the porous microbeads. In this regard, porous microbeads coated with polyethylene glycol (PEG) or porous microbeads coated with polyvinylpyrrolidone (PVP) are preferable, and porous microbead coating with polyvinylpyrrolidone (PVP) is more preferable.

In an exemplary embodiment, the porous microbeads may be, but are not limited to, microbeads including one or more selected from the group consisting of mesoporous silica, styrene, C1-C4-alkyl-substituted styrene, vinylnaphthalene, vinylanthracene, cellulose, styrene-divinyl-benzene copolymer, polyethersulfone (PES), polyethylene (PE), polypropylene (PP), polyvinyl chloride (PVC), polyurethane (PUR), polyethylene terephthalate (PET), polystyrene (PS), acrylonitrile butadiene styrene (ABS), polymethyl methacrylate (PMMA), polytetrafluoroethylene (PTFE), expanded polystyrenes (EPS), polycarbonate (PC), polyamide (PA), epoxy, unsaturated polyester (UP), melamine-formaldehyde (MF), and combinations thereof. Groups of suitable C1-C4-alkyl-substituted styrenes may include, but are not limited to, ethylvinylbenzene, vinyltoluene, diethylstyrene, ethylmethylstyrene or dimethylstyrenes. Any of the various positional isomers of each recited vinyl aromatic monomer may be understood to be suitable. For example, the porous microbeads may be mesoporous silica microbeads; however, porous microbeads exhibiting the aforementioned properties such as pore volume, surface area, and ceria nanoparticle loading capacity may be used without limitation.

In another aspect, the present disclosure relates to an extracorporeal hemopurification cartridge including the porous microbeads.

In an exemplary embodiment, the porous microbeads have a volume fraction of at least 50%, at least 55%, at least 60%, at least 65%, or at least 70%, or at most 90%, at most 85%, or at most 80%, relative to the total extracorporeal hemopurification cartridge. For example, the porous microbeads may have a volume fraction of 50% to 90% relative to the total extracorporeal hemopurification cartridge, but are not limited thereto. Extracorporeal hemopurification and removal of reactive oxygen species may be effectively performed at the above volume ratio.

In another aspect, the present disclosure relates to an extracorporeal hemopurification device including the extracorporeal hemopurification cartridge.

In yet another aspect, the present disclosure relates to an extracorporeal hemopurification method including the steps of:
(a) contacting blood isolated from a subject with the porous microbeads;
(b) removing reactive oxygen species from the blood using the porous microbeads; and
(c) collecting blood from which the reactive oxygen species have been removed.

In an exemplary embodiment, the reactive oxygen species may be removed for a period of 2 to 24 hours during the removing reactive oxygen species, but is not limited thereto, and the porous microbeads are capable of continuous removal of the reactive oxygen species by using a catalyst (ceria nanoparticles) rather than an adsorbent. Thus, the removal of the reactive oxygen species is continuously possible for the time required for the subject without time constraints.

In yet another aspect, the present disclosure relates to a method for preparing porous microbeads for extracorporeal hemopurification , including the steps of (a) synthesizing the porous microbeads and modifying their surface, and (b) attaching ceria nanoparticles to the surface of the modified porous microbeads.

In an exemplary embodiment, the step (a) may modify the surface of the porous microbeads using a formulation containing one or more functional groups selected from the group consisting of amine, thiol, and carboxylic groups.

In an exemplary embodiment, the method may further include (c) coating the surface of the porous microbeads to which the ceria nanoparticles are attached with a polymer.

In an exemplary embodiment, in step (c), the porous microbeads may prepare, but are not limited to, one or more selected from the group consisting of porous microbeads coated with Alginate, porous microbeads coated with polyethylene glycol(PEG), porous microbeads coated with polyvinylpyrrolidone (PVP), and combinations thereof.

### Best Mode for Carrying out the Invention

Hereinafter, the present disclosure will be described in more detail with reference to examples. It will be apparent to those skilled in the art that these examples are merely illustrative of the invention and that the scope of the invention should not be construed as limited by these examples.

### [Example 1]

### Experimental Materials and Methods

### (1-1) Experimental Materials

Mesitylene, ammonium fluoride, poly(ethylene glycol)-block-poly(propylene glycol)-block (ethylene glycol) (Pluronic^{®} P-123) Mn^{~} 5,800, hydrochloric acid, nitric acid, alginic acid sodium salt derived from brown algae, sodium bicarbonate, tetraethyl orthosilicate, 6-aminohexanoic acid, ethyl alcohol, isopropyl alcohol, and acetone were purchased from Sigma-Aldrich (St. Louis, Mo., USA), and cerium (III) nitrate hexahydrate was purchased from Alfa Aesar. Methoxy PEG-amide succinimidyl glutarate 5000 was purchased from SUNBIO CO., LTD (Anyang, Korea). All reagents were used as received without further purification.

### (1-2) Synthesis of Millimeter-Sized Porous Silica Microbeads

Porous silica microbeads were synthesized by a silica sol-gel method using an organic template. Specifically, (1) 16 g of Pluronic^{®} P-123, 80 mL of HCl, 4 mL of 46 mg/mL ammonium fluoride, and 12 g of TMB were dissolved in 516 mL of DI water and stirred at 50 °C for 4 hours. Then, 36.8 mL of TEOS was added to the mixture and reacted for 20 hours. Subsequently, the synthesized porous silica particles were subjected to a hydrothermal reaction at 100 °C for 24 hours, after which the resulting particles were filtered and dried. (2) 10 mL of 20 mg/mL alginate solution and 10 mL of 20, 60, and 100 mg/mL porous silica particles was mixed and injected into a 100 mM CaCl₂ solution through a syringe pump using an electrospray method with a high voltage of 12 kV to synthesize porous silica/alginate microbeads. Subsequently, a silica precursor obtained by reacting 17 mL of 0.15 M HNO₃ and 68 mL of TEOS for 1 hour was mixed with porous silica/alginate microbeads and allowed to react for 24 hours. After the reaction, the collected microbeads were washed with IPA and dried, then calcined at 550 °C for 6 hours to remove alginate and P123, thereby creating pores and ultimately obtaining porous silica microbeads.

### (1-3) Synthesis of Ceria Nanoparticles

1.3117 g of 6-aminohexanoic acid and 70 µL of HCl were dissolved in 60 mL of DI water and heated to 95 °C. Next, 1.08557 g of cerium (III) nitrate hexahydrate was added to the mixture and reacted for 1 minute. The synthesized ceria nanoparticles were precipitated in 500 mL of acetone, washed by centrifugation three times at 10000 rpm for 10 minutes, and then dried in a vacuum oven for 24 hours. The dried ceria nanoparticles were redispersed in DI water and stored at 4 °C until use.

### (1-4) Preparation of Ceria Nanoparticles/Porous Silica Microbeads

Since the surface of the porous silica microbeads is (-) charged, while the ceria nanoparticles are (+) charged, it is possible to produce ceria nanoparticles/porous silica microbeads by inducing electrostatic bonding by polarity difference. 1 g of porous silica microbeads is mixed with 20 mL of ceria nanoparticle solution at a concentration of 5 mg/mL and reacted for 24 hours. Subsequently, the mixture was washed three times with deionized water and three times with acetone, then collected and dried in a vacuum oven for 24 hours. The loading efficiency of the ceria nanoparticles was calculated by measuring the amount of ceria nanoparticles present in the supernatant via ICP-MS measurement and the difference from the amount of mixed ceria nanoparticles.

### (1-5) Preparation of PEG-Coated Ceria Nanoparticles/Porous Silica Microbeads

For polyethylene glycol (PEG) coating of porous silica microbeads 500 mg of methoxy PEG-amide succinimidyl glutarate 5000 was dissolved in sodium bicarbonate buffer pH 8.0 and 1 g of ceria nanoparticles/porous silica microbeads was added to the mixture and mixed for 24 hours. After the reaction, the PEG-coated ceria nanoparticles/porous silica microbeads were washed three times with deionized water and stored at 4 °C until use.

### (1-6) Preparation of PVP-Coated Ceria Nanoparticles/Porous Silica Microbeads

1 g of polyvinylpyrrolidone (PVP) was dissolved in deionized water and mixed with 1 g of ceria nanoparticles/porous silica microbeads and reacted for 24 hours. After the reaction, the PVP-coated ceria nanoparticles/porous silica microbeads were washed three times with deionized water and stored in deionized water at 4 °C until use.

### (1-7) Characteristics Analysis of Porous Silica Microbeads

The surface morphology and elemental analysis of all microbeads were examined by SEM and EDS (JSM 7000F, JEOL, Japan). Pore size, pore volume and surface area were measured using the Brunaurer-Emmett-Teller (BET) method.

### (1-8) Analysis of in vitro ROS Removal Ability of Microbeads

10, 25, and 50 mg of porous silica microbeads and ceria nanoparticle/porous silica microbeads were mixed with a 500 µM hydrogen peroxide (H₂O₂) solution. After 2 hours, 50 µL of the supernatant was collected and mixed with 50 µL of a working solution containing 100 µM Amplex Red reagent and 0.2 U/mL horseradish peroxidase (HRP), and allowed to react at room temperature for 30 minutes. The reaction product resorufin exhibits fluorescence with an excitation wavelength of 545 nm and an emission wavelength of 590 nm using a microplate reader (Varioskan LUX, Thermo, Massachusetts, USA). The hydroxyl radical antioxidant capacity of porous silica microbeads and ceria nanoparticles/porous silica microbeads were measured by HORAC Activity Assay Kit. 10 mg of porous silica microbeads and ceria nanoparticle/porous silica microbeads were prepared in a 1.5 mL tube. 40 µL of deionized water, 280 µL of fluorescein probe, 40 µL of hydroxyl radical initiator, and 40 µL of Fenton reagent were added to the porous silica microbeads and the ceria nanoparticles/porous silica microbeads, and the mixture was incubated at room temperature for 60 minutes with slight shaking. A 200 µL of sample solution was collected and measured at excitation 480 nm and emission 530 nm every 5 minutes for a total of 1 hour (Varioskan LUX, Thermo, Massachusetts, USA). All measurements were performed at least three times.

### (1-9) Analysis of in vitro ROS Removal Ability Using Microbeads of Cartridge System

A cartridge with a volume of about 1 mL was fabricated, and the top and bottom of the cartridge were equipped with 8 mm mesh filters to prevent particles from escaping. 200 mg of porous silica microbeads, ceria nanoparticles/porous silica, PVP-coated ceria nanoparticles/porous silica microbeads were prepared in the fabrication cartridge. A 2 mM hydrogen peroxide (H₂O₂) solution was added to the top using a syringe pump at a 1 mL/min flow rate for 10 minutes. After 10 minutes, a 50 µL sample was transferred to a 96-well plate and the remaining H₂O₂ solution was injected back into the cartridge at 1-, 2-, 4- and 7-time intervals. The removal efficiency of hydrogen peroxide is calculated using the same method (Amplex Assay Kit).

### (1-10) Blood Adsorption Test for Microbeads

The blood adsorption test was performed by placing 10 mg of porous silica microbeads, ceria nanoparticles/porous silica microbeads, PVP-coated ceria nanoparticles/porous silica microbeads in 10 mL of blood and incubating at 37 °C for 2 hours.

### (1-11) Analysis of Treatment Effect Using Microbeads of Cartridge System

A 5 mg/kg dose of endotoxin (Lipopolysaccharide; LPS) is intravenously infused in laboratory rats to induce sepsis. Catheters are inserted into the carotid artery, femoral artery and femoral vein, and hemopurification circuits, cartridges, perfusion pumps are connected. For the control, the cartridge is excluded. The perfusion pump is operated at a rate of 1.5 ml per minute to allow extracorporeal blood circulation from 10 minutes after sepsis induction. After sepsis induction, 30 ml/kg of physiological saline is intravenously injected, and when the mean arterial pressure decreases to less than 60 mmHg, norepinephrine, which is a blood pressure increasing agent, is injected at a rate of up to 1 meg/kg/min. Extracorporeal blood circulation ends after 4 hours. Observe and record for 4 hours whether the laboratory rats are dead, mean arterial pressure, and amount of vasopressor used. In the experiment of measuring the blood lactate level and the leukocyte-releasing hydrogen peroxide level, blood samples are collected at 1 hour and compared in order to eliminate the skewing caused by the death of the subject.

### [Example 2]

### Characteristics Analysis Results of Porous Silica Microbeads

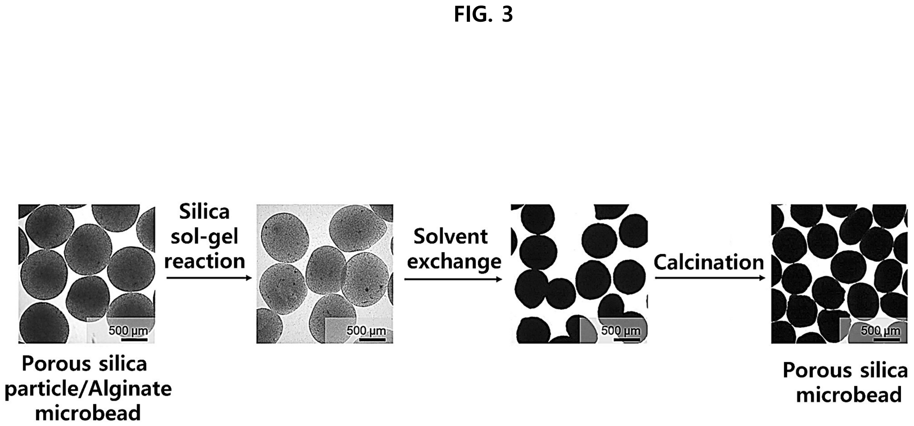

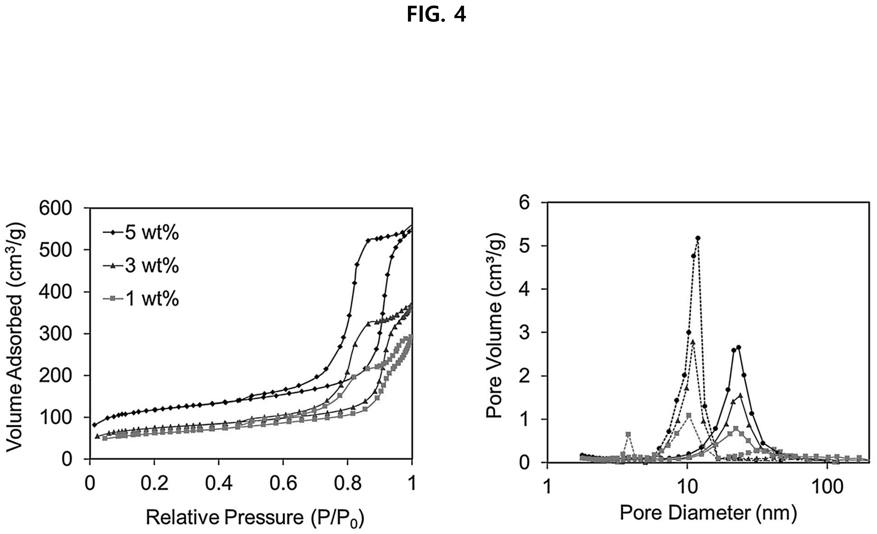

Porous silica microbeads were fabricated by synthesizing alginate hydrogel microbeads containing porous silica microparticles therein and using them as a template (FIG. 3). The porous silica/alginate hydrogel was then subjected to a silica sol-gel reaction to induce bonding between the porous silica microparticles, followed by calcination to remove the organic alginate and P123 to finally obtain porous silica microbeads. The alginate hydrogel microbeads without porous silica particles are greatly reduced in size and distorted in shape after undergoing the same process. However, alginate hydrogel microbeads containing porous silica microparticles are observed to be of similar size after calcination. Porous silica microbeads were fabricated by varying the concentration of porous silica microparticles included in the alginate hydrogel to control the porosity of the porous silica microbeads (FIG. 4). The surface area and pore volume of porous silica microparticles varied with concentration: at 1 wt%, the surface area was 207.33 m²/g and the pore volume was 0.45 cm³/g; at 3 wt%, the surface area was 244.73 m²/g and the pore volume was 0.56 cm³/g; at 5 wt%, the surface area was 395.17 m²/g and the pore volume was 0.84 cm³/g. Thus, it was found that the porosity (surface area and pore volume) increased as the concentration of the included porous silica microparticles increased. While high porosity was preferable in the experiment, the alginate solution containing 5 wt% of porous silica had a viscosity that was too high, leading to low productivity, so a concentration of 3 wt% porous silica was used in subsequent experiments.

### [Example 3]

### Loading Efficiency of Ceria Nanoparticles of Prepared Ceria Nanoparticle/Porous Silica Microbeads

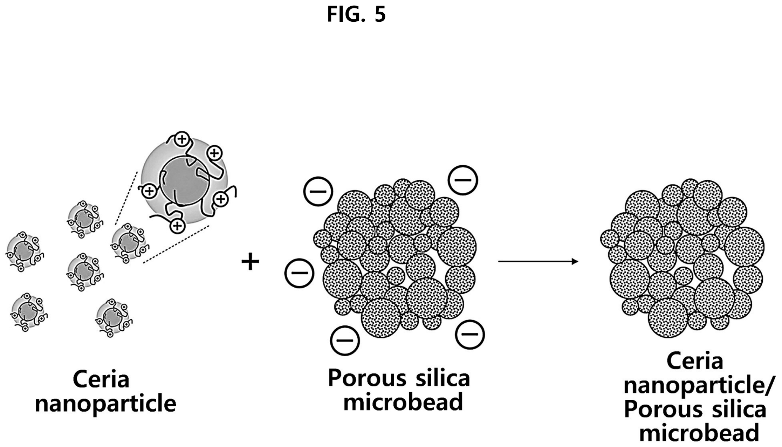

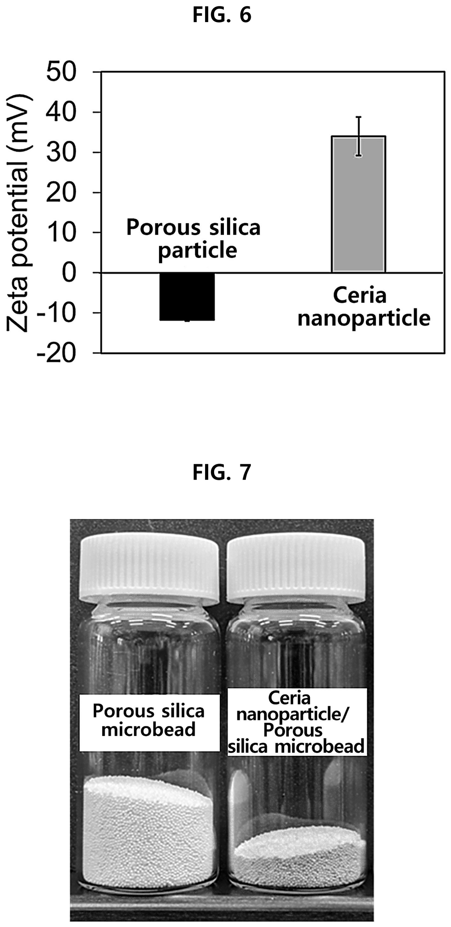

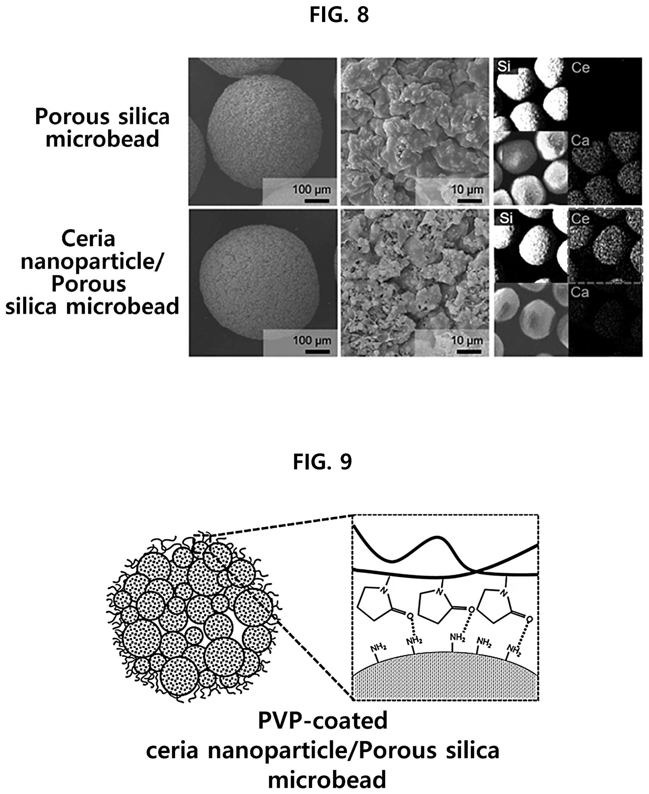

The ceria nanoparticles have a positive surface charge and may be readily absorbed onto the negatively charged surface of the porous silica microbeads (FIGS. 5 and 6). A mixture of 1 g of porous silica microbeads and 5 mg/mL ceria nanoparticles was prepared in 20 mL to facilitate the adsorption of ceria nanoparticles onto the porous silica microbeads. As a result, ceria nanoparticles/porous silica microbeads whose color was slightly changed to yellow by the loaded ceria nanoparticles were obtained (FIG. 7). In addition, ICP-MS analysis of the supernatant (remaining ceria nanoparticles) after mixing the ceria nanoparticles and the porous silica microbeads showed a loading efficiency of 100%. When observed via SEM, the surface of the porous silica microbeads appeared to be a combination of uneven and smooth clusters, while the surface of the ceria nanoparticles/porous silica microbeads exhibited a rough texture throughout the clusters. Energy-dispersive X-ray Spectroscopy (EDS) confirmed that the amount of elemental Ce was detected in the ceria nanoparticles/porous silica microbeads, indicating adsorption of the ceria nanoparticles (FIG. 8).

### [Example 4]

### Analysis of Ceria Nanoparticles/Porous Silica Microbeads Coated with PVP for Hemocompatibility

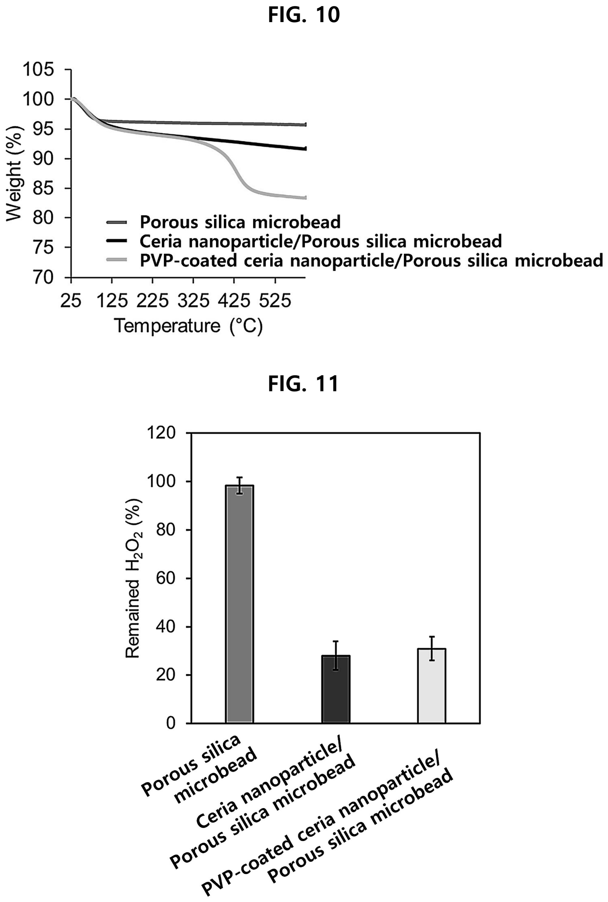

PVP was coated onto ceria nanoparticles/porous silica microbeads via physical adsorption for hemocompatibility (FIG. 9). 1 g of ceria nanoparticles/porous silica microbeads was mixed with 20 mL of a 50 mg/mL PVP aqueous solution and reacted for 24 hours, followed by washing with deionized water 5 times and then collecting, and the fabricated PVP-coated ceria nanoparticles/porous silica microbeads were dispersed in deionized water and stored in a refrigerator. Thermogravimetric analysis (TGA) confirmed that the synthesized PVP-coated ceria nanoparticles/porous silica microbeads were coated with PVP at about 8% weight ratio (FIG. 10).

### [Example 5]

### Results of Determination of ROS Removal Ability of PVP-coated Ceria Nanoparticles/Porous Silica Microbeads

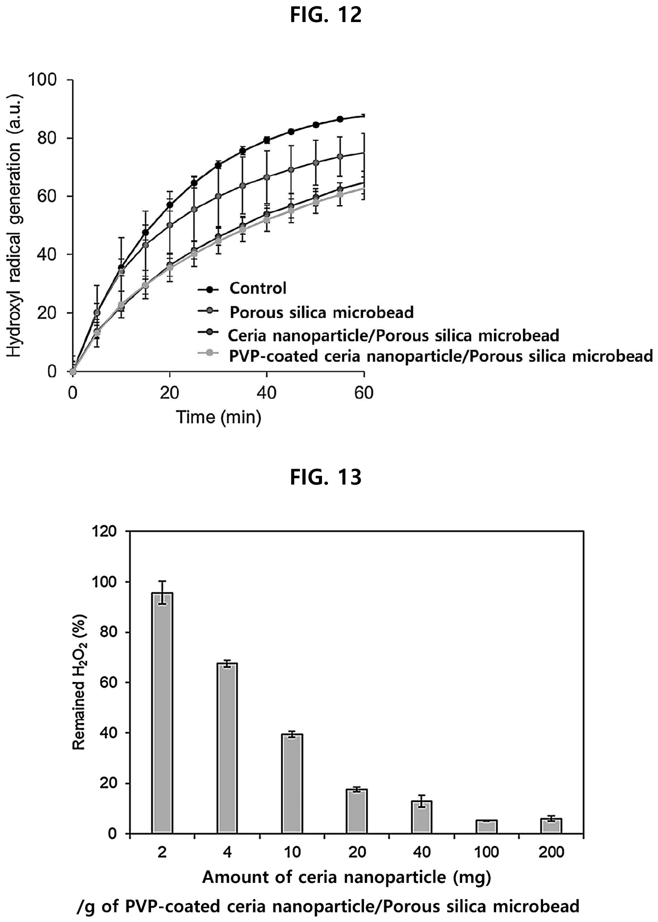

The hydrogen peroxide (H₂O₂) and hydroxyl radical analysis using the porous silica microbeads not loaded with ceria nanoparticles, the ceria nanoparticles/porous silica microbeads, and the PVP-coated ceria nanoparticles/porous silica microbead showed that there was almost no removal of H₂O₂ by the porous silica microbeads not loaded with ceria nanoparticle, but both microbeads loaded with ceria nanoparticles had sufficient ROS removal ability (FIGS. 11 and 12). This means that ROS removal ability remains undiminished even after PVP coating.

When PVP-coated ceria nanoparticles/porous silica microbeads loaded with 2, 4, 10, 20, 40, 100, and 200 mg of ceria nanoparticles per 1 g of microbeads, respectively, were reacted in 2 mL of 500 µM H₂O₂ for 1 hour to confirm the amount of ceria nanoparticles required for porous microbeads having sufficient ROS removal properties, it was confirmed that higher reactive oxygen species (hydrogen peroxide) removal ability was exhibited as the amount of the ceria nanoparticles increased (FIG. 13). The PVP-coated ceria nanoparticles/porous silica microbeads loaded with 2 mg/g of ceria nanoparticles showed almost no removal of H₂O₂, whereas those with 4 mg/g of ceria nanoparticles exhibited approximately 30% removal efficiency, those with 10 mg/g showed about 50% removal efficiency, and those with 20 mg/g demonstrated over 80% removal efficiency. Since ceria nanoparticle loading of 100 mg or more exhibited over 90% removal ability of reactive oxygen species with little difference in removal ability, PVP-coated ceria nanoparticle/porous silica microbeads containing 100 mg of ceria nanoparticles per 1 g of microbeads were fabricated for use in cartridge fabrication in subsequent experiments.

### [Example 6]

### Results of Determination of ROS Removal Ability of Ceria Nanoparticles/Porous Silica Microbeads in a Cartridge for Hemopurification

Whether the ceria nanoparticles/porous silica microbeads could effectively operate in the cartridge to remove ROS was confirmed by creating a practical use environment.

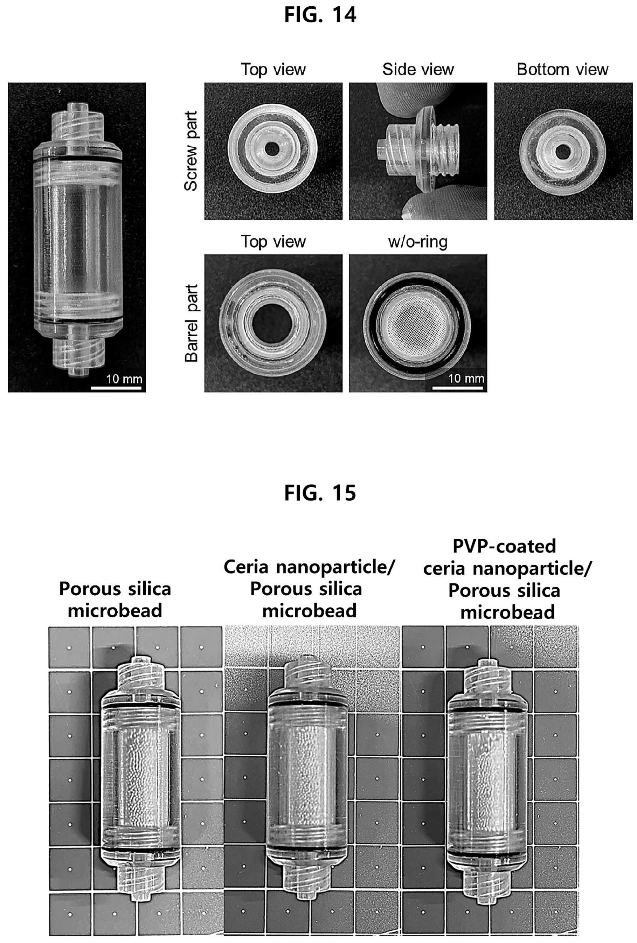

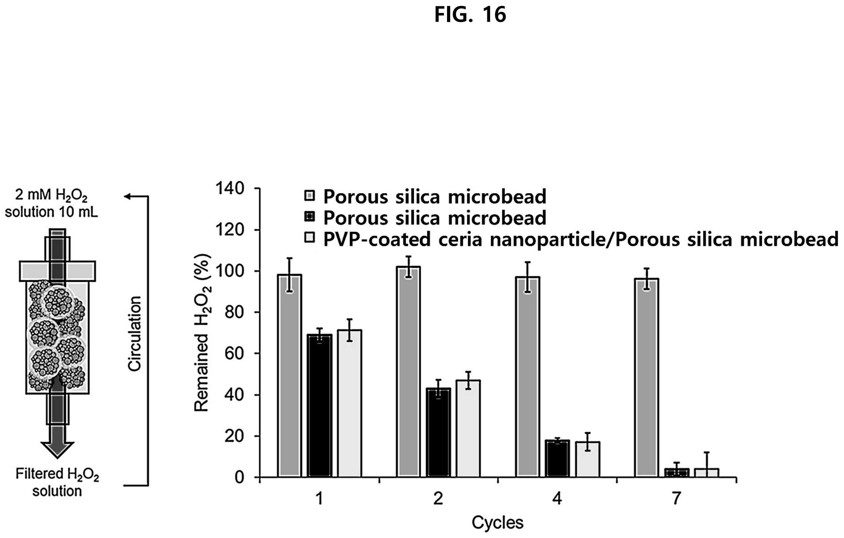

FIG. 14 shows photographs of each part of the cartridge fabricated by a 3D printer, as well as the fully assembled cartridge. A cartridge for removing reactive oxygen species in blood was fabricated by placing 200 mg of porous microbeads inside the fabricated cartridge. As an example, cartridges containing porous silica microbeads, ceria nanoparticles/porous silica microbeads, and PVP-coated ceria nanoparticles/porous silica microbeads were fabricated (FIG. 15). To test the ROS removal ability of each cartridge, perfusion and collection were performed repeatedly with 10 mL of 2 mM H₂O₂ solution, and the ROS removal efficiency was measured for 7 cycles (FIG. 16). As a result, the porous silica microbeads were unable to remove H₂O₂, whereas the ceria nanoparticles/porous silica microbeads showed removal efficiencies of about 40% after one cycle, about 50% after two cycles, and about 80% and 95% after four and seven cycles, respectively. The PVP-coated ceria nanoparticles/porous silica microbeads also exhibited a similar ROS scavenging effect as the ceria nanoparticles/porous silica microbeads despite being coated with the PVP polymer. That is, it was confirmed that excellent catalase-mimicking activity was maintained even after PVP coating.

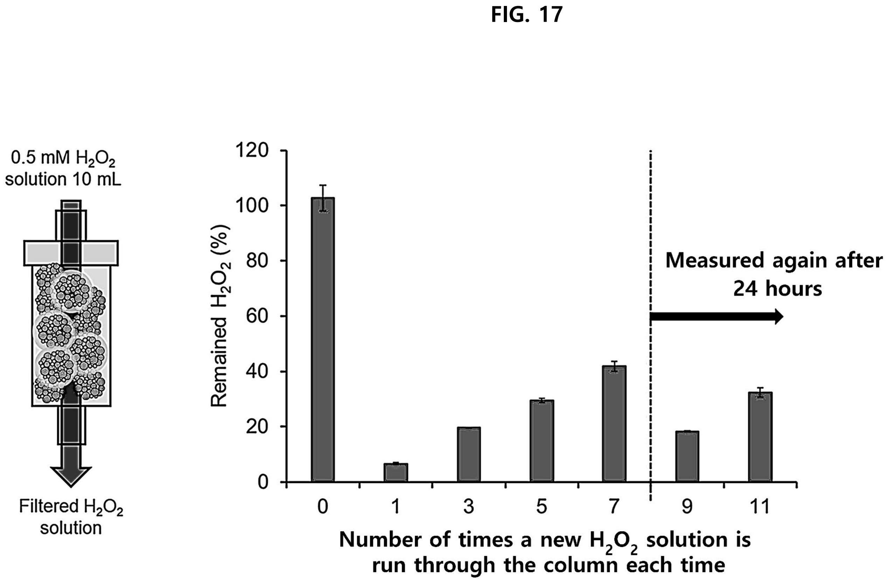

Unlike the above experiment in which the hydrogen peroxide solution passed through the cartridge was collected, and the cartridge flowed again, the ROS removal efficiency was measured for 7 cycles after passing a new hydrogen peroxide solution through the cartridge each time, and as a result, even a high concentration of hydrogen peroxide solution showed a removal rate of 60% or more until 7 cycles (FIG. 17). In addition, after repeating the experiment 7 times, we waited 24 hours and passed the hydrogen peroxide solution through the cartridge again 8, 9, 10, and 11 times. It was confirmed that the catalase-mimicking activity was restored again even when the hydrogen peroxide solution was passed through the cartridge for 8 to 11 cycles after 24 hours.

This indicates that ceria nanoparticles/porous silica microbeads and PVP-coated ceria nanoparticles/porous silica microbeads may potentially eliminate ROS in future external blood cartridge systems.

### [Example 7]

### Comparison of ROS Removal Ability of Porous Microbeads and Glass Microbeads without Pores in a Cartridge for Hemopurification

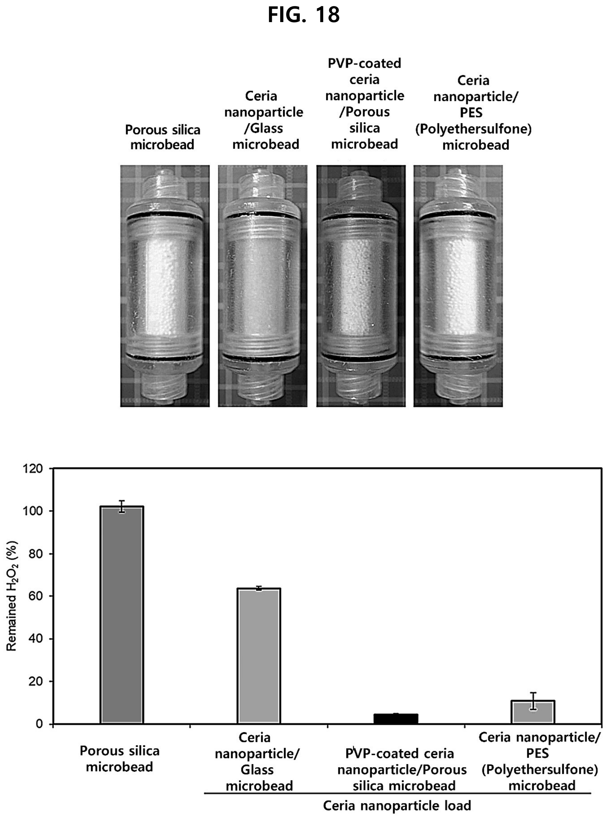

The catalase mimicking properties of cartridges fabricated by loading ceria nanoparticles onto nonporous glass microbeads and porous PES(polyethersulfone) polymer microbeads were compared with those of PVP-coated ceria nanoparticle/porous silica microbead cartridges (FIG. 18). Unlike microbeads not loaded with ceria nanoparticles, which do not remove hydrogen peroxide, cartridges fabricated with all microbeads loaded with ceria nanoparticles showed hydrogen peroxide removal properties. However, in the case of glass microbeads without pores, a certain level of hydrogen peroxide was removed, but the efficiency was lower than that of other porous microbeads (about 40%), and when the composition of the porous microbeads was changed to a PES polymer, the catalase-mimicking property was maintained at about 90%, which was confirmed to be similar to the 95% level of the porous silica microbead-based cartridge. This means that porous microbeads may load a large amount of ceria nanoparticles and thus have superior removal ability of reactive oxygen species compared to non-porous microbeads with the same capacity.

### [Example 8]

### Blood Adsorption Analysis Results

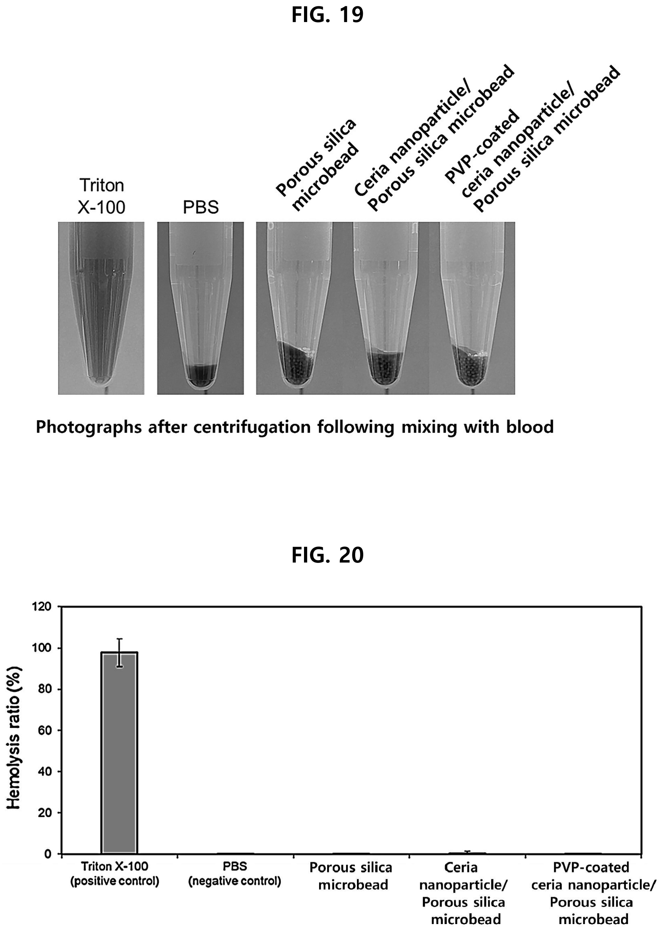

Erythrocytes in the blood may be easily hemolyzed or aggregated by reaction with external substances. For use in the bloodstream, the microbeads for hemopurification should not react with or destroy erythrocytes. After mixing 10 mg of porous silica microbeads, ceria nanoparticles/porous silica microbeads, and PVP-coated ceria nanoparticles/porous silica microbeads with 1 mL of erythrocyte solution and allowing them to react for 2 hours, the microbeads were separated by centrifugation, and the absorbance of the supernatant was measured to analyze hemolyzed erythrocytes, resulting in all tested microbeads showing no hemolytic properties (FIGS. 19 and 20).

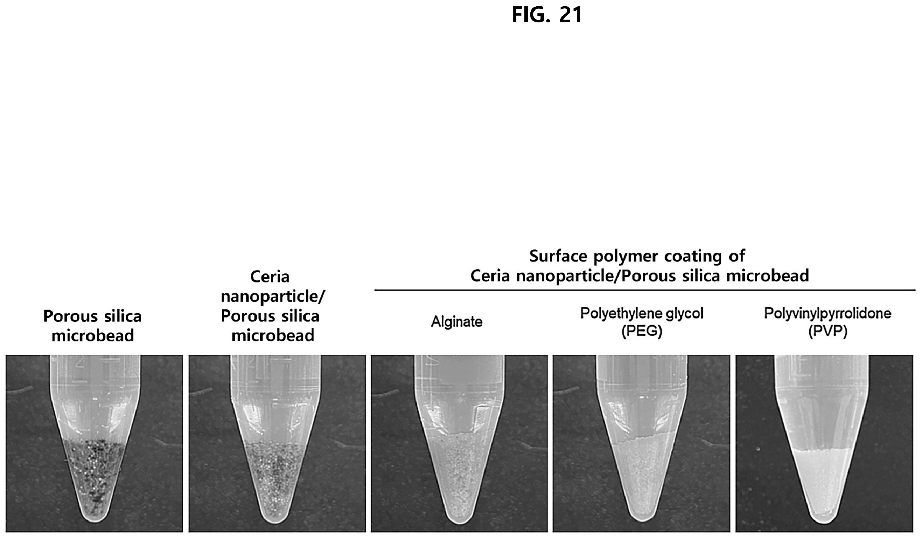

In order to confirm the degree of adsorption of the microbeads to the erythrocytes in the blood, 100 mg of each of the porous silica microbeads, the ceria nanoparticles/porous silica microbeads, and the ceria nanoparticles coated with alginate, PEG, or PVP/porous silica microbeads was incubated with 10 mL of blood at 37 °C for 2 hours and then centrifuged, and the microbead was observed after washing with PBS three times (FIG. 21). As a result, the porous silica microbeads and the ceria nanoparticle/porous silica microbeads exhibited red color due to adsorption of erythrocytes, while the ceria nanoparticles coating with alginate and PEG/porous silica microbeads exhibited a relatively low level of erythrocyte adsorption. However, the PVP-coated ceria nanoparticle/porous silica microbeads exhibited their original color with almost no adsorption of erythrocytes, indicating that blood adsorption did not occur. That is, PVP is considered the most suitable polymer coating material to prevent the adsorption of blood onto the microbeads.

### [Example 9]

### Evaluation of the Therapeutic Effect of PVP-coated Ceria Nanoparticles/Porous Silica Microbeads in a Cartridge for Hemopurification

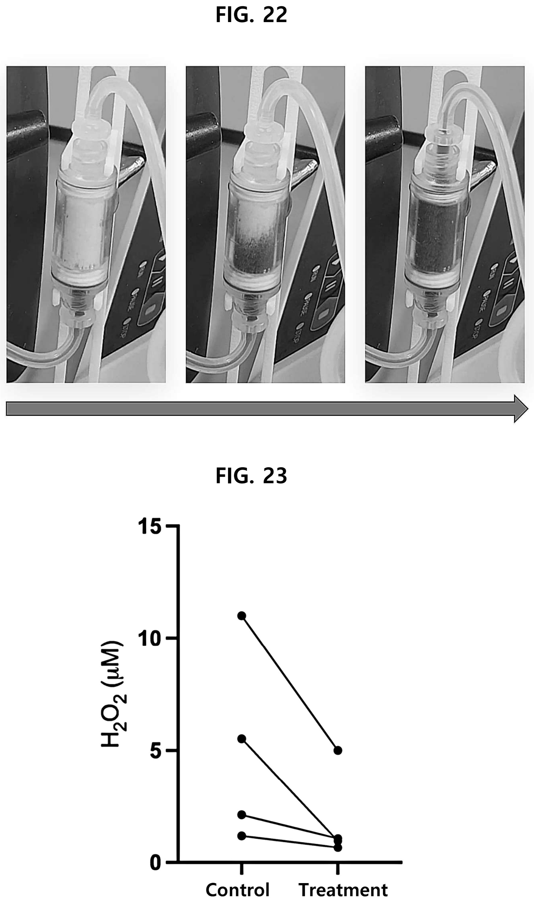

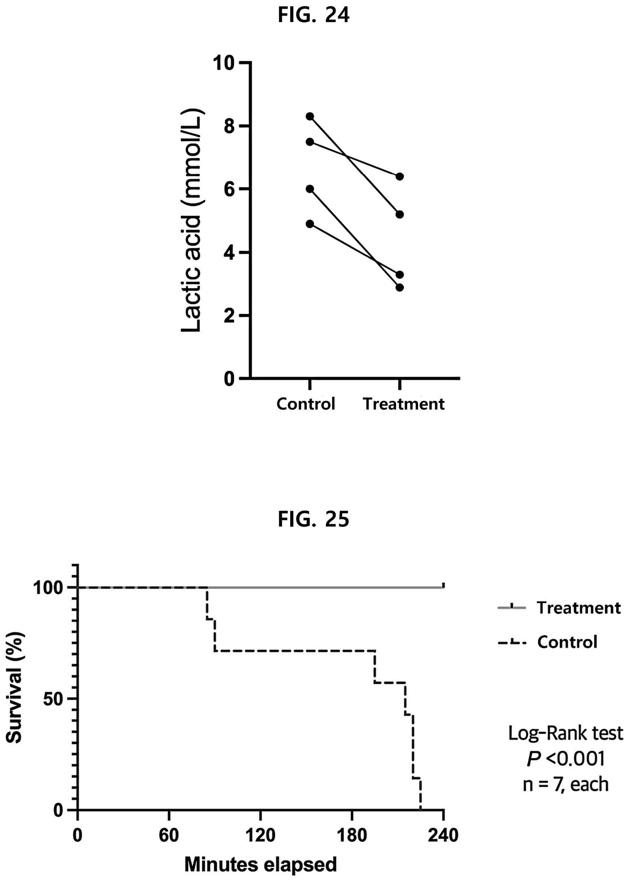

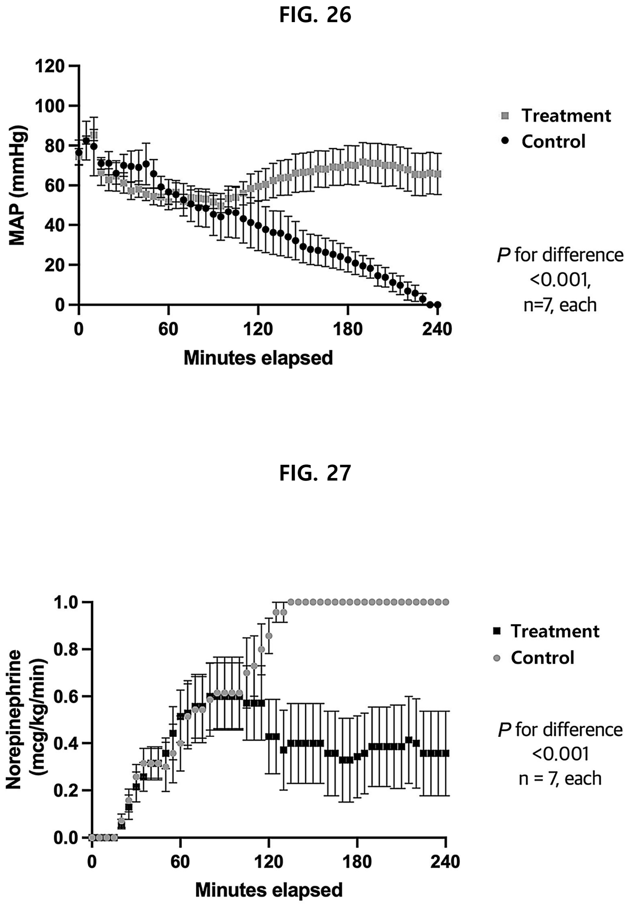

As a result of extracorporeal hemopurification in rats in which sepsis was induced by LPS intravenous infusion (FIG. 22), the group subjected to extracorporeal hemopurification treatment had a statistically significantly lower concentration of hydrogen peroxide released by leukocytes than the control group (FIG. 23), and the blood lactate levels indicating the severity of septic shock were significantly decreased (FIG. 24). The control group had 100% mortality (n=7 of 7), whereas the group receiving extracorporeal hemopurification treatment showed a significant difference in survival rate of 0% mortality (n=0 of 7) (FIG. 25). The extracorporeal hemopurification treatment group showed a significantly higher mean arterial pressure (MAP) level than the control group (FIG. 26), and the amount of vasopressor used was also low (FIG. 27).

## Claims

1. A porous microbead for extracorporeal hemopurification comprising a ceria nanoparticle.

2. The porous microbead of claim 1,
wherein the porous microbead remove reactive oxygen species through the catalytic action of the ceria nanoparticle.

3. The porous microbead of claim 1,
wherein the porous microbead has a size (diameter) of 1 to 1,500 µm.

4. The porous microbead of claim 1,
wherein the porous microbead has a pore volume of 0.1 cm³/g to 13.6 cm³/g.

5. The porous microbead of claim 1,
wherein the porous microbead has a surface area of 100 m²/g to 1,000 m²/g.

6. The porous microbead of claim 1,
wherein the porous microbead comprise at least 3 mg/g of the ceria nanoparticle.

7. The porous microbead of claim 1,
wherein the porous microbead is at least one selected from the group consisting of porous microbeads coated with Alginate, porous microbeads coated with polyethylene glycol (PEG), porous microbeads coating with polyvinylpyrrolidone (PVP), and combinations thereof.

8. The porous microbead of claim 7,
wherein the porous microbead is a porous microbead coated with polyvinylpyrrolidone (PVP).

9. An extracorporeal hemopurification cartridge comprising the porous microbead of any one
of claims 1 to 8.

10. The extracorporeal hemopurification cartridge of claim 9,
wherein the porous microbead has a volume fraction of 50% to 90% relative to the total extracorporeal hemopurification cartridge.

11. An extracorporeal hemopurification device comprising the extracorporeal hemopurification cartridge of claim 9.

12. An extracorporeal hemopurification method, comprising:
(a) contacting blood isolated from a subject with porous microbeads of any one of claims 1 to 8;
(b) removing reactive oxygen species from the blood using the porous microbeads; and
(c) collecting the blood from which the reactive oxygen species have been removed.

13. A method of preparing the porous microbead for extracorporeal hemopurification of any one of claims 1 to 8, comprising:
(a) synthesizing the porous microbead and modifying its surface; and
(b) attaching the ceria nanoparticle to the modified surface of the porous microbead.

14. The method of claim 13,
wherein the step (a) modifies the surface of the porous microbeads using an agent containing one or more functional groups selected from the group consisting of amine, thiol, and carboxylic groups.

15. The method of claim 13, further comprising (c) coating a polymer on the surface of the
porous microbead to which the ceria nanoparticle is attached.
